(19)

Europäisches
Patentamt
European
Patent Office
Office européen
des brevets

(11) **EP 3 834 832 A1**

(12) **EUROPEAN PATENT APPLICATION**
published in accordance with Art. 153(4) EPC

(43) Date of publication:
**16.06.2021 Bulletin 2021/24**

(21) Application number: **19845974.5**

(22) Date of filing: **17.07.2019**

(51) Int Cl.:
*A61K 35/10* (2015.01)    *A23L 33/10* (2016.01)
*A61P 9/12* (2006.01)    *A61P 43/00* (2006.01)

(86) International application number:
**PCT/JP2019/028115**

(87) International publication number:
**WO 2020/031638 (13.02.2020 Gazette 2020/07)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **10.08.2018   JP 2018159754**

(71) Applicant: **Kohaku Bio Technology Co., Ltd.
Ibaraki 3050006 (JP)**

(72) Inventors:
• **TAKEDA, Reiko**
**Tsukuba-shi, Ibaraki 305-0006 (JP)**
• **HAEIWA, Haruna**
**Tsukuba-shi, Ibaraki 305-0006 (JP)**
• **YAMAMOTO, Takuya**
**Tsukuba-shi, Ibaraki 305-0006 (JP)**
• **YAMANO, Mikio**
**Tsukuba-shi, Ibaraki 305-0006 (JP)**

(74) Representative: **Schön, Christoph
Dr. Schön, Neymeyr & Partner mbB
Bavariaring 26
80336 München (DE)**

(54) **ANGIOTENSIN I-CONVERTING ENZYME ACTIVITY INHIBITOR**

(57)    Provided is an ACE activity inhibitor that can be integrated into easy-to-take daily food and drink. The ACE activity inhibitor of the present invention contains an amber extract wherein an extraction solvent thereof is hydrous ethanol (moisture content: 1 mass% to 60 mass%).

FIG.1

1.  $5^4$-fold dilution
2.  $5^5$-fold dilution
3.  $5^4$-fold dilution
4.  $5^3$-fold dilution
5.  $5^2$-fold dilution
6.  $5$ -fold dilution

mean±SD,(N=3)

EP 3 834 832 A1

## Description

Technical Field

The present invention relates to an angiotensin I-converting enzyme activity inhibitor, comprising an amber extract.

Background Art

Amber is a fossil formed by lying a resin of principally a pine plant underground for a long time and condensing the resin. In China, a powder of amber has long been used as a Chinese medicine. Amber primarily contains a resin, an essential oil and succinic acid, and is slightly soluble in ethanol and diethyl ether or benzene (see, for example, Non Patent Literature 1).

Amber is well known as jewelry in Japan. Recently, powder and an extract of amber have been increasingly used in cosmetics and healthy foods. For example, a technique for blending amber power with a cosmetic to improve touch to the skin (see, for example, Patent Literature 1); a technique for blending an amber extract with an external preparation to skin (see, for example, Patent Literature 2 and Patent Literature 3); a technique for using the whitening effect of an amber extract (see, for example, Patent Literature 4 and Patent Literature 5); a technique for using a skin turnover promoting factor contained in an amber extraction fraction (see, for example, Patent Literature 6); a technique for using a skin firming effect of an amber extract (see, for example, Patent Literature 7); a technique for using a hyaluronic acid production-promoting factor in an amber extraction fraction (see for example, Patent Literature 8); and a technique for using an angiogenesis-promoting factor contained in an amber extraction fraction (see, for example, Patent Literature 9) are known.

As described above, amber is known to have various physicochemical and biological effects and has been used in a wide variety of fields as an extremely useful material. Amber is expected as a material having unlimited potential.

As one of the mechanisms for regulating blood pressure, a renin-angiotensin system is known. Angiotensin I-converting enzyme (ACE) is an enzyme producing a substance responsible for increasing blood pressure, i.e., angiotensin II, from angiotensin I, in the renin-angiotensin system and considered as a key factor for preventing hypertension. Recently, for preventing and improving hypertension, functional foods such as foods for specified health uses and foods with function claims have been marketed. In particular, a component of food having an ACE activity inhibitory effect is frequently investigated.

As those having such an ACE activity inhibitory effect, e.g., methylated catechin (see, for example, Patent literature 10) and a peptide such as a proteolytic product (see, for example, Patent literature 11, Patent literature 12) are known; however, a new and more effective material has been continuously desired in the market.

Citation List

Patent Literatures

Patent Literature 1: Japanese Patent Laid-Open No. 2004-83478
Patent Literature 2: Japanese Patent Laid-Open No. H9-227334
Patent Literature 3: Japanese Patent Laid-Open No. 2001-131048
Patent Literature 4: Japanese Patent Laid-Open No. 2010-235551
Patent Literature 5: Japanese Patent Laid-Open No. 2012-240967
Patent Literature 6: Japanese Patent Laid-Open No. 2007-314522
Patent Literature 7: Japanese Patent Laid-Open No. 2008-189669
Patent Literature 8: Japanese Patent Laid-Open No. 2008-266260
Patent Literature 9: Japanese Patent Laid-Open No. 2011-256164
Patent Literature 10: Japanese Patent Laid-Open No. 2011-79789
Patent Literature 11: Japanese Patent Laid-Open No. 2009-51813
Patent Literature 12: Japanese Patent Laid-Open No. 2008-297208

Non Patent Literatures

Non Patent Literature 1: Chinese Medicine Basic Dictionary, volume 2, Shanghai Science and Technology Publisher (Jiangsu New Medical School, "Chinese Medicine Basic Dictionary" Editorial Department, edited by Shogakukan)

Summary of Invention

Technical Problem

An object of the present invention is to provide an ACE activity inhibitor that can be integrated into easy-to-take daily food and drink.

Solution to Problem

In consideration of the circumstance, the present inventors conducted intensive studies. As a result, they found that an amber extract has an ACE activity inhibitory effect, which has not been known. Based on the finding, the present invention was achieved. The present invention is as follows.

<1> An angiotensin I-converting enzyme activity inhibitor comprising an amber extract wherein an extraction solvent thereof is hydrous ethanol (moisture content: 1 mass% to 60 mass%).
<2> An oral administration composition for inhibiting an angiotensin I-converting enzyme activity, prepared by blending the angiotensin I-converting enzyme activity inhibitor according to <1>.
<3> The oral administration composition for inhibiting an angiotensin I-converting enzyme activity according to <2>, wherein the aspect is a pharmaceutical product, a quasi-drug, a food and drink or a food additive.

Advantageous Effects of Invention

The ACE activity inhibitor of the present invention comprising an amber extract can inhibit production of a substance increasing blood pressure, i.e., angiotensin II.

Brief Description of Drawings

FIG. 1 is a graph showing inhibition of angiotensin I-converting enzyme activity by the amber extract of Production Example 1.
FIG. 2 is a graph showing diastolic blood pressure and systolic blood pressure by intake of the amber extract of Production Example 2.

Description of Embodiments

<The amber extract of the present invention>

The ACE activity inhibitor according to the present invention contains an amber extract as an active ingredient. The amber extract herein refers to, e.g., amber itself, processed amber such as crushed or chopped amber, an amber extract with a solvent added to amber or processed amber, a solvent-free amber extract obtained by removing the solvent from the amber extract, and purified products of these. Of them, an amber extract or a solvent-free amber extract is particularly preferable. Also, the amber to be used in the present invention is not particularly limited by the production area; however, taking production and reserve thereof into consideration, amber produced in Kaliningrad of Russia is preferable.
Examples of the solvent for the amber extract include water, an alcohol such as methanol, ethanol, 1,3-butanediol, propylene glycol and glycerin; an ester such as ethyl acetate and methyl formate; a nitrile such as acetonitrile; an ether such as diethyl ether and tetrahydrofuran; a halogenated hydrocarbon such as chloroform and methylene chloride; and a ketone such as acetone and methyl ethyl ketone. These solvents may be used singly or as a mixture (of two or more). Of these solvents, water or an alcohol is preferable and hydroalcoholic solution is more preferable. As the alcohol, ethanol having a water content of 1 mass% to 60 mass%, and preferably 10 mass% to 50 mass% can be used. If the water content deviates from the upper lower end of the range mentioned above, extraction efficiency may deteriorate.
An extraction method is as follows. For example, to crushed amber, a solvent in a volume 2 to 20 times of the amber is added. If the extraction is carried out at room temperature, the crushed amber may be soaked for several days; whereas, if the extraction is carried out at about the boiling point, the crushed amber may be soaked for several hours. Thereafter, the resultant extract is subjected to filtration to remove insoluble matter. The filtrate may be concentrated under vacuum. The concentrate may be purified by column chromatography using a column packed with silica gel, octadecylsilyl silica gel or ion exchange resin.

<Production Example 1>

Powder (100 g) of amber produced in Kaliningrad of Russia was extracted with 50% ethanol (ethanol having a water content of 50%), concentrated under vacuum and lyophilized to obtain a 8 g of a 50% ethanol extract.
Examples of drinks containing the extract of the present invention include tea drinks, coffee drinks, soft drinks, alcohol drinks, milk drink, carbonated drinks, healthy drinks, nutrition drinks, sports drinks and concentrated stock solutions of these and preparation powders. Examples of food include gums, candies, jellies, tablets, healthy food, nutritional supplementary food and supplements.
When the extract of the present invention is used as a medicine such as a prophylactic agent for hypertension, the extract is provided in the dosage form of a powder, a granule, a tablet, a capsule, a liquid and an injection. The extract of the present invention can be orally administered directly or as a dilution of the extract with water. Alternatively, the extract of the present invention may be prepared into a preparation in combination with a pharmaceutical carrier known in the art. More specifically, the extract of the present invention can be administered as an oral liquid preparation such as a syrup or as an oral solid preparation, such as tablets, capsules, granules and powders, which is prepared by processing the extract of the present invention into a liquid extract or a powder and blending the liquid extract or power with a pharmaceutically acceptable carrier. As the pharmaceutically acceptable carrier, an organic or inorganic carrier substance ordinarily used as preparation material is used and blended as an excipient, a lubricant, a binder and a disintegrant in a solid preparation, and as a solvent, an excipient, a suspending agent and a binder in a liquid preparation. If necessary, additives such as a preservative, an antioxidant, a coloring and a sweetener can be used in the preparations.
In a food and drink or a pharmaceutical composition containing the extract of the present invention, the extract of the present invention can be added in any concentration. The extract of the present invention is preferably added in a concentration of 0.01 to 50 mass% and more preferably 0.05 to 20 mass% of the total amount of a food and drink or a pharmaceutical composition.
The effective dosage can be appropriately determined depending on the age and body weight of a patient, the type and severity of the disease, and the administration route.
Now, the present invention will be more specifically described by way of Examples below; however, the present invention is not limited to these Examples.

Examples

<Example 1> ACE activity inhibition test

ACE activity was measured in accordance with the following procedure by using ACE Kit-WST (trade name, manufactured by DOJINDO LABORATORIES):

(1) The amber extract (10 mg) of Production Example 1 was dissolved in dimethyl sulfoxide (1 ml). After centrifugation, the supernatant was diluted serially with pure water 5 times. The individual dilutions were used as sample solutions.
(2) The sample solutions (sample) or pure water (blank 1, blank 2) were added to individual wells of a 96 well plate each in an amount of 20 $\mu$l.
(3) To individual wells, a substrate buffer was added in an amount of 20 $\mu$l.
(4) To the well of blank 2, pure water was added each in an amount of 20 $\mu$l.
(5) To each of the wells to which the sample solution was added and the well of blank 1, the enzyme solution (20 $\mu$l) was added. The plate was subjected incubation to be performed at 37°C for 60 minutes.
(6) To each well, a color-producing liquid was added in an amount of 200 $\mu$l, and the plate was subjected incubation to be performed at room temperature for 10 minutes.
(7) Absorbance at 450 nm was measured by a plate reader.
(8) ACE inhibitory activity (inhibition rate %) is calculated in accordance with the following expression.

$$\text{ACE inhibitory activity (inhibition rate \%)} = [(A_{blank1} - A_{sample})/(A_{blank1} - A_{blank2})] \times 100$$

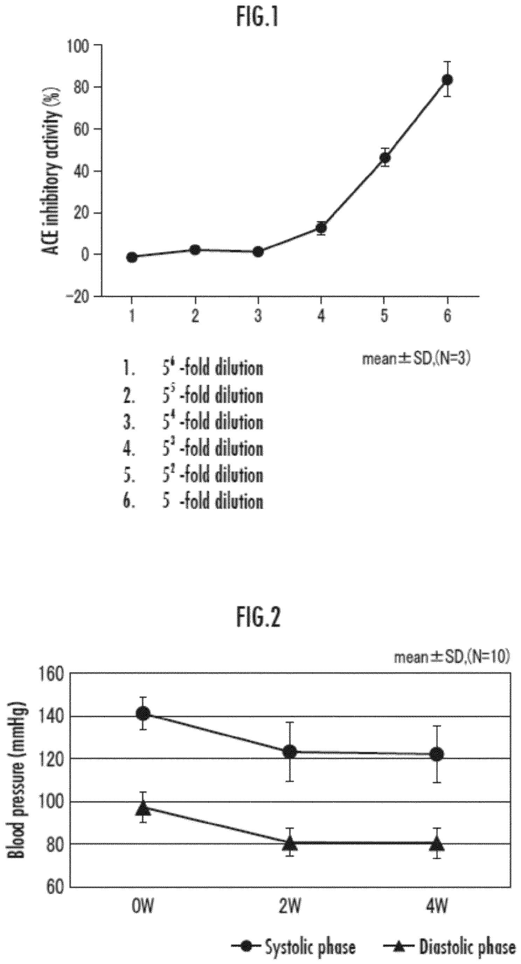

From the results shown in FIG. 1, it was confirmed that the amber extract of the present invention inhibits ACE activity in a concentration dependent manner.

<Example 2> Effect of amber extract on blood pressure

The subjects (10 persons) having high blood pressure were allowed to take an amber extract (100 mg) of Production Example 1 once a day for four weeks. Blood pressure (systolic phase and diastolic phase) were measured before intake (0 W), 2 weeks (2 W) after intake and 4 weeks (4 W) after intake.

From the results of FIG. 2, it was confirmed that the amber extract of the present invention facilitates a decrease of both systolic blood pressure and diastolic blood pressure.

**Claims**

1. An angiotensin I-converting enzyme activity inhibitor comprising an amber extract wherein an extraction solvent thereof is hydrous ethanol (moisture content: 1 mass% to 60 mass%).

2. An oral administration composition for inhibiting an angiotensin I-converting enzyme activity prepared by blending the angiotensin I-converting enzyme activity inhibitor according to claim 1.

3. The oral administration composition for inhibiting an angiotensin I-converting enzyme activity according to claim 2, wherein an aspect is a pharmaceutical product, a quasi-drug, a food and drink or a food additive.

## FIG.1

1. $5^6$ -fold dilution
2. $5^5$ -fold dilution
3. $5^4$ -fold dilution
4. $5^3$ -fold dilution
5. $5^2$ -fold dilution
6. $5$ -fold dilution

mean±SD,(N=3)

## FIG.2

mean±SD,(N=10)

—●— Systolic phase    —▲— Diastolic phase

## INTERNATIONAL SEARCH REPORT

| International application No. |
| --- |
| PCT/JP2019/028115 |

A. CLASSIFICATION OF SUBJECT MATTER
Int.Cl. A61K35/10(2015.01)i, A23L33/10(2016.01)i, A61P9/12(2006.01)i,
A61P43/00(2006.01)i

According to International Patent Classification (IPC) or to both national classification and IPC

B. FIELDS SEARCHED

Minimum documentation searched (classification system followed by classification symbols)
Int.Cl. A61K35/10, A23L33/10, A61P9/12, A61P43/00

Documentation searched other than minimum documentation to the extent that such documents are included in the fields searched
Published examined utility model applications of Japan 1922-1996
Published unexamined utility model applications of Japan 1971-2019
Registered utility model specifications of Japan 1996-2019
Published registered utility model applications of Japan 1994-2019

Electronic data base consulted during the international search (name of data base and, where practicable, search terms used)
WPI, JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
| --- | --- | --- |
| A | JP 2011-57556 A (IWATE UNIV) 24 March 2011, claim 1, paragraph [0002] (Family: none) | 1-3 |
| A | CN 101468188 A (WU, L.) 01 July 2009, claim 1 (Family: none) | 1-3 |
| P, A | WO 2018/212362 A1 (YAMANOBEAUTYCHEMICAL INC.) 22 November 2018 (Family: none) | 1-3 |

| ☐ Further documents are listed in the continuation of Box C. | ☐ See patent family annex. |
| --- | --- |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search 21.08.2019 | Date of mailing of the international search report 03.09.2019 |
| --- | --- |
| Name and mailing address of the ISA/ Japan Patent Office 3-4-3, Kasumigaseki, Chiyoda-ku, Tokyo 100-8915, Japan | Authorized officer Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004083478 A
- JP H9227334 B
- JP 2001131048 A
- JP 2010235551 A
- JP 2012240967 A
- JP 2007314522 A

- JP 2008189669 A
- JP 2008266260 A
- JP 2011256164 A
- JP 2011079789 A
- JP 2009051813 A
- JP 2008297208 A

**Non-patent literature cited in the description**

- Chinese Medicine Basic Dictionary. Jiangsu New Medical School, ''Chinese Medicine Basic Dictionary. Shanghai Science and Technology Publisher, vol. 2